# EUROPEAN PATENT SPECIFICATION

(11) **EP 0 632 895 B1**
(45) Date of publication and mention of the grant of the patent: **08.07.1998**
(21) Application number: 93908606.2
(22) Date of filing: 26.03.1993
(51) Int. Cl.: G01N 35/00, G01N 33/543, G01N 33/546

(54) **RAPID HYPERSENSITIVE FLOWTHROUGH IMMUNODETECTION SYSTEM**
SCHNELLES HOCHEMPFINDLICHES IMMUNANALYTISCHES DURCHFLUSS-NACHWEISSYSTEM
SYSTEME D'IMMUNODETECTION PAR ECOULEMENT RAPIDE ET HYPERSENSIBLE

(30) Priority: 27.03.1992 US 859164
(43) Date of publication of application: 11.01.1995
(73) Proprietor: PERSEPTIVE BIOSYSTEMS, INC., Cambridge, MA 02139 (US)
(72) Inventor: AFEYAN, Noubar, B., Brookline, MA 02146 (US); DORVAL, Brent, Douglas, MA 02139 (US); REGNIER, Fred, E., West Lafayette, IN 47906 (US)
(74) Representative: Beetz & Partner Patentanwälte
(86) International application number: US9302845
(87) International publication number: WO9320449

(56) References cited:
- EP-A- 0 449 321
- EP-A- 0 456 308
- WO-A-91/00762
- WO-A-92/18636
- US-A- 4 128 628
- US-A- 4 816 418
- BIOTECHNIQUES vol. 11, no. 2, 1991, pages 226 - 231 S. P. FULTON ET AL. 'ANTIBODY QUANTITATION IN SECONDS USING AFFINITY PERFUSION CHROMATOGRAPHY.'

## Description

### Field of the Invention

This invention relates generally to methods and apparatus for conducting assays for analytes. In particular, the invention relates to methods and apparatus for conducting highly sensitive specific binding assays for analytes utilizing a novel insoluble matrix comprising binding sites for the analytes.

### Background of the Invention

The need to rapidly detect the presence or concentration of trace quantities of organic compounds in various industrial, research, and clinical contexts is well understood in the art. Millions of clinical assays have been conducted. Immunoassay is now exploited to detect the presence of toxins or contaminants in food and water, and in many other research, diagnostic, and quality control contexts. Process monitoring systems are of particular importance in the production of therapeutic substances for human use, where Federal regulatory agencies place stringent controls on product quality. The monitoring system should be rapid, allowing the data generated to be used in a feedback system to adjust process parameters. The monitoring system should also be adaptable and easily altered to analyze different samples. Tests which are sensitive, fast, easy to perform, and readily adaptible for assay of a given antigen are in great demand.

High performance liquid chromatography (HPLC), high performance capillary electrophoresis (HPCE), rapid analytical immunoassay (RAIA), or some combination of these, are alternative analytical techniques that may be adapted for rapid process monitoring. Each of these techniques has limitations. Chromatography and electrophoresis analyze samples by separation, based on size or charge. During the early stages of protein purification the product may be contaminated with a large number of other proteins and constitute less than 10% of the total protein mass, making it difficult to identify a protein of interest in a process stream by conventional separation techniques. Similarly, at the very late stages of protein purification, protein purity is very high, generally exceeding 95%. At high concentrations of a single product, a chromatogram or electropherogram is essentially a single peak, making it difficult to detect small concentrations of contaminants in this range. Separation methods here are also of limited utility.

In addition, chromatographic and electrophoretic analyses are limited by the time required to perform them. The speed of conventional HPLC analysis generally is limited by the porous particulate chromatographic packings used in conventional HPLC. While mobile phase mass transfer between the particles is convectional in HPLC, mobile phase mass transfer within the porous particles is primarily diffusional, substantially slowing the progress of the protein through the matrix. The speed of electrophoresis separation is a function of the voltage applied across the system. However, heat production increases with the square of the voltage. Thus, the maximum voltage most electrophoresis systems will tolerate is limited by the speed with which they can dissipate the heat generated by the applied voltage.

Of the three alternative analytical techniques, rapid analytical immunoassay (RAIA) appears to be the only technique of sufficient discriminating power to isolate a protein of interest from both crude samples and substantially pure products. Moreover, because an immunoassay involves a specific binding interaction with the protein of interest, to a first approximation, the assay provides a method of determining if the protein is folded correctly. However, conventional analytical immunoassays, performed as equilibrium reactions, also are limitec by the incubation times required in most assays.

Immunoassays are based on the well-known reactions between an antibody or other specific binding protein and the specific antigen or hapten (analyte) for that antibody. The general principal can be applied to any specific binding interaction between a binding protein and its ligand. In the "competitive" protocol, an analog of the sample antigen or analyte competes with the sample antigen for complex formation with an antibody which typically is immobilized on an insoluble support. The competing analog is often a purified form of the sample antigen that is tagged with a detectable moiety, e.g., with a fluorescent or radioactive tracer material. The analog also can be tagged with an enzyme having a readily assayable activity, such as reacting with a substrate to give a colored product. The fraction of tagged analog bound to the antibody (or remaining free in solution) is then measured, and is related to the amount of sample antigen (analyte) present in the reaction. It has generally proven easier to quantitate the bound species.

Many approaches to this technique have been developed which differ mostly in the order and timing of the addition of sample antigen and tagged analog to the antibody, and in the method of separating bound and free species. (See Bolton et al., Handbook of Experimental Immunology, Weir, D.M., Ed., Blackwell Scientific Publications, Oxford, 1986; Vol. 1, chapter 26, for a general discussion on immunoassays.)

Immobilized immunoassays (called immunosorbent assays) wherein the antibodies are immobilized on a solid support of the type often used in chromatography, such as Sepharose beads, also are known. The Sepharose beads can be packed into a column, allowing the sample to be captured, and impurities to be washed away. The antibody (or immobilized component) is generally covalently attached to the solid Sepharose support. Alternatively, the antibody may interact reversibly with the solid support if the support's surface includes a component, such as protein A, capable of specific, reversible interaction with the antibody. If the immobilized component is reversibly bound to the solid support, the bound complexes can be eluted and the analog-antibody complexes quantitated. Quantitation requires that the analog be tagged in some way, to distinguish complexed analog from complexed sample antigen. See, for example, Mattiasson et al., Proc. Int. Symp. on Enzyme-Labelled Immunoassay of Hormones and Drugs, Pal, S., Ed., Walter de Gruyter, Berlin (1978), p. 91.

Biotechniques, Vol. 11, No. 2, 1991, pages 226-231, discloses an assay technique for antibodies using protein A or protein G bound to Perfusion Chromatography™ support matrices.

If the immobilized component is covalently attached to the solid support, the sample antigen can be analyzed by simply eluting it from the column and quantitating it with a UV detector, potentially eliminating the need for a tagged competitor. Unfortunately, many compounds used to elute sample antigens, so-called eluting agents, coelute with the antigen and can cause a significant perturbation of the detection base line, making it difficult to differentiate the elution profile of the sample antigen from that of the eluting agent. In practice, this effect can diminish the sensitivity of the assay by more than 100 fold, depending on the detection wavelength and eluting agent used. A second disadvantage with this system, and of particular importance for a rapid monitoring system, is that it typically requires covalent immobilization of the binding protein or antibody to prevent its elution with the antigen. This means that the column must either be dedicated to the analysis of a single antigen for its lifetime, or that the column be repacked each time a different sample antigen is to be sampled.

An additional concern with conventional analytical immunoassays as a monitoring protocol is that the requirement for equilibrium conditions often demands substantial incubation times. In fact, U.S. Patents Nos. 4,816,418 and 4,128,628 each describe an apparatus for the automated assay of multiple samples in sequence that attempt to overcome the limitation imposed by reaction incubation times. In each apparatus the samples can be sidetracked to incubate and are retrieved to quantify after the reactions have reached equilibrium. A typical assay may take ten or more minutes to perform, which is often too long for feedback analysis.

A second specific binding assay protocol involves formation of a binding site-analyte-tagged binding site complex known as a "sandwich." The most popular form of this assay is the enzyme linked immunosorbent assay (ELISA). In an ELISA, a first antibody (Ab1) targeted against a specific antigen (Ag) is immobilized on a solid support, producing an immunosorbent (support-Ab1). In research, the most widely used immunosorbent is a polystyrene plate, or a microtiter plate. The microtiter plate has a series of wells, and antibodies are immobilized on the walls of the wells. The ELISA method involves incubation of the immunosorbent with a solution suspected to contain the antigen to form a sorbed antibody antigen complex (support-Abl:Ag). The sorbed antigen is then incubated with a second antibody (Ab2) to which an enzyme (E) has been conjugated to form a support Ab1:Ag:Ab2-E complex or "sandwich". The support-Ab1:Ag:Ab2-E complex is then incubated with enzyme substrate. The reaction of the enzyme with the substrate then may be quantitated by absorbance, fluorescence, or other detection means. Antigen quantitation is achieved by relating the rate of product formation, or the total amount of product formed in a fixed time, to the amount of absorbed antigen.

ELISA determinations generally are carried out on a fixed time basis, and can only span approximately a 50 fold range of concentration, without changing either incubation time or some other variable. This results in the problem that samples of unknown antigen concentration may be too dilute or too concentrated for the ELISA system. When the sample is too concentrated, one must use multiple dilutions to bring the sample into the range of the assay. On the other hand, when the sample is too dilute, one must use longer incubation times. Both of these approaches are time consuming. ELISA determinations generally require 1-24 hours. Because the assay is relatively slow, it is necessary to carry out large numbers of assays in parallel to obtain sufficiently high throughput to be useful.

Antibodies, antigens and enzymes are immobilized at a surface in the ELISA systems, and therefore the enzyme assay portion of the determination is heterogeneous. The rate of transfer of substrate to the surface and product away from the surface can be rate limiting. This is particularly true when the amount of sorbed enzyme is high. Substrate will be exhausted near the surface and product will build up to higher concentrations at the surface than in the rest of the reaction vessel. This is a particularly significant problem with enzymes that are product inhibited. Product inhibition will cause the immunological assay to be non-linear.

The ELISA system also suffers from the problem of nonspecificity. When the concentration of antigen in the solution being assayed is very low, only a few of the Ab1 binding sites are occupied by Ag. In this case it is possible for some of the Ab2-E conjugate to sorb to the immunosorbent non-specifically. Ab2-E conjugate that is non-specifically sorbed will also produce product and give a signal higher than expected from the amount of Ag absorbed.

De Alwis and Wilson reported that antibodies immobilized on a chromatographic material could be used in a continuous flow ELISA system, wherein the products of the immobilized enzyme reaction were formed continuously and transported to a detector. (de Alwis, W.U. and Wilson, G.S. (1985) Anal. Chem. 57 pp. 2754-2756.)

It is an object of this invention to provide a method and apparatus for the rapid, quantitative assay of an analyte that can be used as part of a protein production monitoring system for all stages of protein purification, and which can provide an alternative to conventional immunoassay methods. Another object of the invention is to provide a method and apparatus for the assay of plural, different samples of analytes. Still another object is to provide a method for the rapid assay of samples of an analyte to overcome the time limitations imposed by conventional immunoassay and separation techniques.

These and other objects and features of the invention will be apparent from the description, drawings, and claims that follow.

### Summary of the Invention

Disclosed is an apparatus and method for the rapid flowthrough assay of an analyte. A binding protein having a binding site capable of binding selectively to an analyte is immobilized on the surface of a rigid matrix defining a flowpath, the flowpath having a flowpath volume fillable with fluid having access to the surface. The geometry of the matrix provides several unique attributes to the method and apparatus. A liquid sample containing the analyte and other reagents used in specific binding assays may be delivered convectively through the matrix, to the flowpath volume, and substantially adjacent the surface comprising the immobilized binding protein to permit formation of a binding protein:analyte complex at a rate not dependent on diffusion. A detectable tagging reagent capable of binding with the binding protein or the complex is then delivered convectively to the matrix. The amount of tagging reagent which is bound thereafter is detected to provide an assay of the concentration or presence of the analyte. The amount of bound tagging reagent may be detected conventionally using a spectrophotometer, a spectrofluorometer, a luminescence detector or other means known in the art. The binding protein/analyte combination can be any receptor/ligand combination capable of specific reversible binding, including antibody and antigen. The apparatus and methods may be used to detect any substance for which a specific binding material is known, can be produced, or be manufactured. The speed, versatility, and sensitivity of the assay methods permit them to be used in process monitoring and quality control procedures. A particularly useful application of the methods is in clinical analyses, to assay the presence and/or concentration of different solutes in a body fluid sample. In this case, different binding proteins may be included on the matrix for each analyte.

The rapid flowthrough assays may be implemented using high speed matrices capable of perfusion. These matrices typically comprise particles which may be of the same overall size as are sometimes employed in conventional chromatography matrices, but having increased intraparticle porosity. Typically, 10-20 µm diameter particles of perfusive matrices have intraparticle throughpores of relatively large mean diameter (e.g., 6000-8000Å) and a network of smaller mean diameter (500-1500 Å) subpores extending from or interconnecting the larger throughpores. The resulting network limits the diffusional path lengths within the particles so that mass transfer within the particle pores is essentially governed by convection. The effect is to increase the mobile phase velocity of these systems to greater than 10-100 times that of conventional HPLC systems (for example, greater than 1000cm/hr), without substantial loss in binding capacity, and in fact effective substantial enhancement. The reduced diffusional pathlengths in the perfusive matrix systems greatly enhances the speed of the assays of this invention.

Combined with the increased porosity of the particles capable of perfusive transport is a substantially increased surface area available for attachment of binding protein, typically to levels within the range of 30 to 50 m²/ml, as measured by mercury porosimetry, for 10-20 µm particles. One ml of perfusive matrix is capable of binding at least about 10-20 mg of binding protein. Thus, for example, 10 µg of binding protein occupies less than 1/1000 of the total binding protein attachment capacity of a one ml matrix. The exceptionally high surface area provided by perfusive matrices allows one to reduce the column volume required for an assay significantly. A binding protein may be immobilized on the matrix through either covalent or noncovalent interactions. The binding protein can be reversibly bound to the matrix surface, to provide a system that is flexible and reusable.

A description of perfusive matrix material is provided in U.S. Patent No. 5,019,270, issued May 28, 1991; and in Afeyan et al., (1990) Bio/Technology 8:203-206. Perfusive matrix materials are available commercially from PerSeptive Biosystems, Inc. of Cambridge, Massachusetts, U.S.A. The preferred material for manufacture of the particles is polystyrene divinylbenzene copolymer.

In addition, a non-porous particulate matrix may be used, wherein tortuous channels are formed by the interstitial space among the particles. Such matrices have a much lower net capacity than perfusive matrices, and require operation at higher pressure drops, but can be used and may be very useful for microanalysis.

Matrices useful in the process and apparatus of the invention may take various forms in addition to the packed particle forms disclosed above. They can comprise one or a bundle of microcapillaries, very shallow beds structurally similar to a membrane, or three dimensional cylindrical structures which are the equivalent of intercontacted packed particles. What the matrices have is common is that 1) reagents can be delivered convectively very close to the active surface comprising the binding protein and 2) the ratio of the area of the surface to the volume that can be filled with liquid adjacent that surface is at least about 10⁴ cm² /cm³ , or 10⁴ cm⁻¹ , preferably 10⁶ cm⁻¹ . While diffusive transport of solute such as analyte, tagged moieties, etc., is always necessary if the solute is to react with a surface bound substance, the distance diffusion must occur is necessarily very small, e.g., no greater than about 10,000Å.

Matrices may be utilized which have a coating on the hydrophobic surface of the matrix beads which produces a continuous hydrophilic film. The coating compounds may contain hydroxyl, epoxy, halide, or other reactive side groups, as described in United States Patent No. 5,030,352, issued July 9, 1991.

In one embodiment of the invention, the binding protein may be an antibody. In the case where the binding protein is an antibody, the matrix surface may comprise an antibody specific to the Fc region of the binding protein antibody (such as immunoglobulins generated cross-species). In addition, protein A or protein G, two proteins known to bind to the Fc region of immunoglobulins may be used. Multiple antibodies may also be attached to the matrix, to provide an assay system for a plurality of analytes.

The invention provides an optimal system for a rapid flowthrough enzyme linked immunoassay (ELISA). In one embodiment, a binding protein is immobilized on a column containing the perfusive matrix particles. An analyte is then perfused through the column containing the immobilized binding protein to form a binding protein:analyte complex on the matrix. A tagging reagent comprising an enzyme linked to an antibody capable of binding with the complex is then perfused through the column, allowing the tagging reagent to bind to the immobilized binding protein:analyte complex. Perfusion of the matrix with an enzyme substrate solution results in the elution of product, which can be detected spectroscopically or by any of number of methods known in the art. This provides a rapid assay of the analyte.

These and other aspects of the invention will be understood more fully by reference to the following detailed description in conjunction with the attached drawings.

### Brief Description of the Drawings:

Figures 1-4 are illustrations of matrix particles used in one embodiment of the invention.

Figure 5 is an illustration of an apparatus of the invention.

Figures 6-8 are graphs illustrating methods of the invention.

Figure 9 is an illustration of a capillary tube matrix used in one embodiment of the invention.

### Detailed Description

In its broadest sense, this invention describes a method and apparatus for the rapid flowthrough assay of an analyte. The process is useful as a monitor to detect protein concentration in a protein production process. The process may also be used for detecting an analyte in a biological fluid sample.

As used herein, the following terms have the following meanings:
"Analyte", as used herein, includes any sample compound to be analyzed that is capable of being bound specifically and reversibly to another compound, herein referred to as the "binding protein". Useful analytes and binding proteins include antigens (or haptens) and antibody binding sites, ligands and receptors, and any other compound combinations capable of specific reversible binding interactions.
Matrix, as used herein, means a solid structure having a surface with an area accessible to an analyte flowed through or by the surface on which a binding protein may be immobilized.

The invention relates to apparatus and methods for the rapid hypersensitive flowthrough assay of an analyte. A binding protein having a binding site, capable of binding selectively to an analyte, is immobilized on a rigid matrix defining a flowpath the matrix having a surface with an area, the flowpath having a flowpath volume fillable with fluid having access to the surface. A liquid sample containing the analyte is delivered convectively to the flowpath volume through the matrix to permit formation of a binding protein:analyte complex. A detectable tagging reagent capable of binding with the binding protein or the complex is then delivered convectively to the matrix and the amount of tagging reagent which is bound is detected to provide an assay of the concentration or presence of the analyte. The amount of bound tagging reagent may be detected by means of a spectrophotometer, a spectrofluorimeter, an illuminescence detector or other means. In an alternative embodiment, the liquid flowthrough of the analyte or the tagging reagent may be temporarily stopped to provide enhanced binding. In another embodiment, the analyte and the tagging reagent may be preincubated together prior to the convective delivery to enhance binding and then the bound analyte and tagging reagent may be delivered convectively to the matrix together.

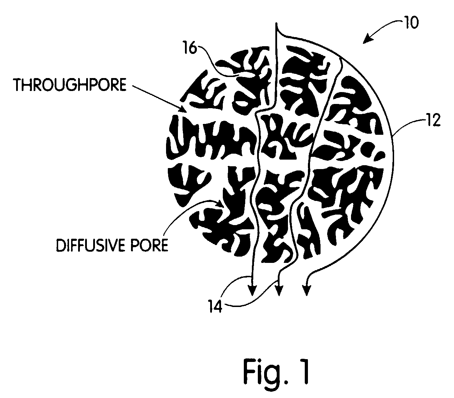

The perfusive matrix suitable for use with the present invention includes close packed particles such as particle 10, illustrated schematically in Figure 1. As is illustrated in Figure 1, the matrix includes a first pore set of extraparticle channels 12, which have a relatively large mean diameter, defined by the interstices among the packed particles. The matrix also includes a second set of throughpores 14 having a smaller diameter and defined by the body of each particle 10. Also defined within particle 10 is a set of diffusive transport pores 16. The mean diameter of the extraparticle channels 12 is larger than that of the throughpores 14. The ratio of the mean diameters of channels 12 and the throughpores 14 is such that, when the particles are close-packed in a bed, there exists a threshold of fluid velocity which can practically be achieved in the bed which induces a convective flow within the throughpores 14 that is faster than the rate of molecular diffusion within the pores. Above the threshold bed velocity, the bed is said to be operating in the perfusive domain where contact between the binding protein and the analyte is no longer bound by diffusive transport. Precisely where this threshold of perfusion occurs depends on many factors, but primarily it depends upon the ratio of the mean diameters of the first and second pore sets. The smaller that ratio, the lower the threshold velocity. In preferred embodiments, the ratio of the mean particle diameter to mean intraparticle throughpore diameter is less than 70, most preferably less than 50.

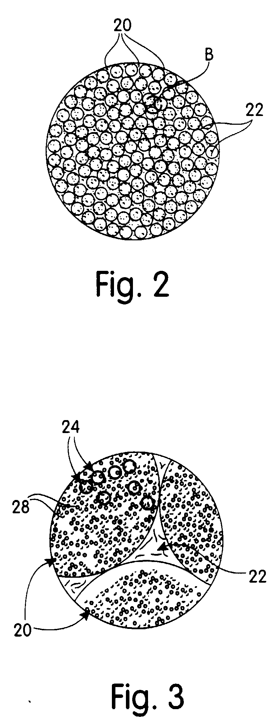

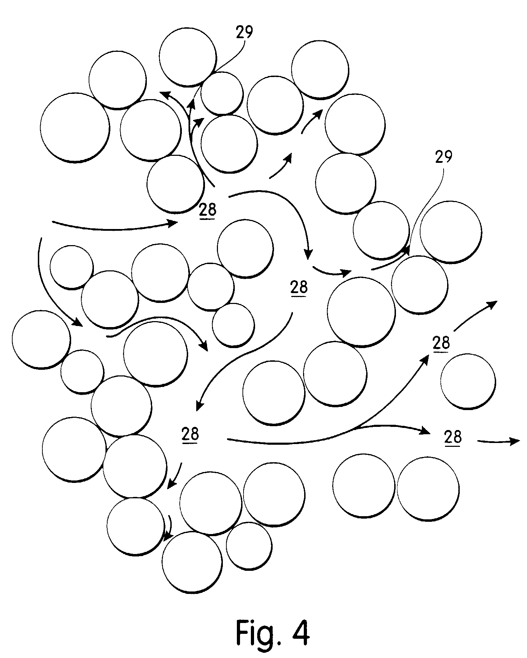

Figures 2 through 4 show a detailed schematic illustration of particles 20, utilized in the perfusive matrix of the invention, in varying scale. Fig. 2 is a cross section of a chromatography column showing a multiplicity of particle 20, approximately 10 µm in diameter, each of which contacts its neighbors and define interstices 22 which comprise the first pore set. The mean diameter of the interstices vary widely but generally will be on the order of 1/3 of the mean diameter of the particles 20. Circle B in Fig. 2 is exploded tenfold in Fig. 3. Here, the microstructure of the bed on a scale of approximately 1 µm is illustrated. The particles comprise clusters of porons illustrated as blank circles 24. The interstices among the poron clusters define throughpores 28. The individual porons making up clusters 24 here are illustrated by dots. At the next level of detail, i.e. 0.1 µm, or 1000Å (not shown) a poron cluster 24 would be seen to comprise a roughly spherical aggregation of porons. In such a structure, the interstices among the porons making up the aggregates 24 are analogous to the diffusively bound particle of conventional chromatography media, in which mass transport is diffusion dependent. Figure 4 illustrates the tortuous path of throughpores 28, within particle 20, through which fluids flow by convection. The perfusive pores are anisotropic, branch at random, vary in diameter at any given point, and lead to a large number of smaller, often blind pores 29 in which mass transport is dominated by diffusion.

Perfusive matrix particles of about 5 to 100 µm diameter may be fabricated from much smaller porons using conventional suspension, emulsion, or hybrid polymerization techniques, from such polymers as styrene cross linked with divinylbenzene or other copolymers, as is disclosed in U.S. Patent No. 5,019,270, issued May 28, 1991.

The perfusive matrix particles include a large surface region onto which binding proteins may be immobilized or other reversible, specific binding structures may be fabricated or attached. Binding proteins can be immobilized on all the surfaces of the particles including the throughpores. Binding proteins may be covalently attached to the surfaces at high concentration using any one of a number of techniques well known to those skilled in the art. Alternatively, binding proteins may be noncovalently attached to the matrix support. Noncovalent attachment provides a number of advantages over covalent attachment, chief among them being cost efficiency and adaptability. A binding protein can be bound to the matrix using any of a number of well-known noncovalent interactions including hydrophobic/hydrophobic interactions. Thereafter the binding protein may be eluted allowing the binding protein and the matrix to be reused.

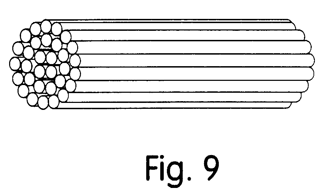

While perfusive matrix materials are preferred, the invention also can be practiced with a non-porous matrix, in which tortuous channels are formed by the interstitial space among non-porous particles. These matrices have a lower net capacity than perfusive matrices but they may be very useful for microanalysis. In addition to packed particles, matrices useful in the process and apparatus of the invention may be embodied as bundles of microcapillaries, as shown schematically in Figure 9. A high surface area/volume ratio may be provided by the use of very small (1-5 µm) internal diameter capillaries, providing a reaction vessel of a few micoliters/cm. The binding protein may be coated on the inner surface of the capillary tube. Solutes are transported through the capillary tube matrix by convection. The high surface area to volume ratio of the capillary tubes increases the available reaction volume. The matrices may further comprise a membrane structure.

In all the matrix materials used in the methods of the invention, the ratio of the area of the surface to the volume that can be filled with liquid adjacent that surface is at least about 10⁴cm⁻¹ , preferably 10⁶cm⁻¹ . In these matrices, reagents can be delivered convectively very close to the active surface comprising the binding sites, so that the distance over which diffusive transport must occur is very significantly diminished in comparison to conventional assay systems.

The matrix provides an exceptionally high surface area to volume ratio (typically on the order of 30-50 m²/ml for 10-20 µm particles) and rapid kinetics. In the perfusion mode, mass transport of solutes including the analyte, tagging reagent or substrate to regions within the matrix is dominated by convection. The distance over which diffusive transport must occur is very significantly diminished. This allows the assay of an analyte using the immobilized binding proteins to be performed in small volumes without compromising capacity. In addition, the rapid throughput of perfusive systems allows the binding protein and analyte as well as the tagging reagent, to be loaded rapidly onto the perfusive matrix (e.g., in seconds or minutes) without loss of binding capacity.

The high surface area matrix, wherein the surface area to liquid fillable volume is at least 10⁴ cm⁻¹ , provides an advantageous system for enhancing the kinetics of an assay. The solutes, including the analyte and tagging reagent are rapidly convectively transported under high pressure through, e.g., a column containing matrix particles having binding proteins immobilized throughout the surface area. The rapid flow through the extensive surface area including channels and pores in the matrix delivers solutes convectively to an enormous excess of binding protein binding sites allowing the solutes to bind rapidly and efficiently to the matrix. Because the diffusional paths of the solution are reduced in the matrix structure, mass transfer is facilitated. As the first binding sites on the matrix are filled, convective flowthrough rapidly delivers solutes to binding sites further down on the column. The kinetics of the assay are enhanced by the rapid flowthrough and the excess of available binding sites.

The high surface area matrices have a very large capacity for binding. For example, 10-20 µm perfusive matrix particles have a surface area typically in the range of 30-50 m²/mL. One mL of perfusive matrix particles is capable of binding 10-20 mg of binding protein. Thus even large (mg) scale assays may be performed using very small amounts of perfusive matrix material.

Immobilized binding sites on the high surface area to volume matrices used herein provide enhanced dynamic range. The concentration of the analyte to be assayed may range from very low (nanogram) quantities to very high (milligram) quantities. When very low quantities of analyte are assayed on the matrix, e.g., a column containing perfusive matrix particles, the analyte will concentrate at the top of the column, at the location of the first available binding sites. In larger scale assays, the analyte can be loaded rapidly onto the column, binding to the first available binding protein sites on the matrix. As the first sites are filled, the convective flow through the perfusive matrix rapidly transports analyte farther down the matrix to fill the next available binding protein sites. Thus both small and large quantities of analyte may be assayed readily and conveniently.

The invention also permits one to immobilize a plurality of antibodies of different types in successive zones on the matrix surfaces to assay for a series of analytes in a single pass. A solution containing the first antibody in the series at a concentration insufficient to saturate all available binding sites on the matrix is provided to the perfusive matrix, e.g., flowed through and among the packed particles in a column. The solution further is provided at a fluid flow velocity sufficient to produce perfusion. At or above this flow rate the antibody will encounter virtually all available binding sites it passes, thereby saturating the first available sites, forming a discrete zone of immobilized antibody. A second solution containing a second antibody, also at a concentration insufficient to saturate all available binding sites, then is added. The antibodies in the second solution, also provided to the matrix at a perfusive flow rate, will flow past the zone of saturated binding sites containing immobilized first antibody, and will begin binding the first available sites encountered further down the column, forming a second zone of immobilized antibody. Other, different antibodies then may be added in the same manner. The number of antibodies that may be added will be limited primarily only by the capacity of the column.

In one important set of embodiments, the invention relates to immunoassay procedures wherein the binding protein is an antibody and the analyte is an antigen. In the case where the binding protein is an antibody, the matrix surface may comprise an antibody specific to the Fc region of the binding protein antibody. In addition, protein A or protein G, two proteins known to bind to the Fc region of immunoglobulins, may be used.

Competitive immunoassays may be performed using the matrices disclosed herein. A sample containing analyte is applied to the matrix, along with a known quantity of a tagged analog of the analyte, such that the analyte and analog compete for complex formation with a limited amount of the immobilized antibody. The competing analog may be a tagged, purified form of the sample antigen. The analog may be tagged, e.g., with a fluorescent or radioactive tracer material, or an enzyme. The fraction of tagged analog bound to the matrix, or the amount which escapes binding, is then measured and is related to the amount of analyte present in the sample. Alternatively, the sample and the tagged analog may be loaded onto the column sequentially. Since competitive assay requires that insufficient binding sites are present to bind all analog and analyte, a matrix having a lower capacity such as non-porous perfusive particles may be advantageous for this method. The amount of labeled analog bound, or escaping binding, may be determined either before or after elution from the column. An enzyme substrate solution passed through the column will be acted on by the immobilized enzyme, and the extent of reaction can be assessed by monitoring reaction production in the effluent.

In the currently preferred form of assay, the analyte is detected by a "sandwich" procedure in which a second antibody binds to previously bound analyte, wherein the second antibody is linked to an assayable moiety such as an enzyme. In the procedure, the rate at which the reagents are brought into interactive contact with the surface is dominated by convection as opposed to diffusion used in the normal enzyme linked immunoassay (ELISA). Two or more assays may be easily conducted simultaneously. The flow of enzyme substrate used to assess the amount of enzyme tag immobilized on the column may be stopped for a short period of time during passage through the column to enhance sensitivity.

In one embodiment of an ELISA, a binding protein is immobilized on a column containing the perfusive matrix particles. An antigen (the analyte) is then perfused through the column containing the immobilized binding protein to form a binding protein:analyte complex on the matrix. A tagging reagent comprising an enzyme linked to an antibody capable of binding with the complex is then perfused through the column, allowing the tagging reagent to bind to the immobilized binding protein:analyte complex. Perfusion of the matrix with an enzyme substrate solution results in the elution of product which can be detected spectroscopically or by any of number of methods known in the art. This provides a rapid assay of the analyte. Any of a number of parameters may be adjusted to improve resolution. The flow rate may be adjusted. The flow also may be stopped after loading solutes such as the analyte, tagging reagent or enzyme substrate to allow the solute to bind or react on the matrix, thereby enhancing binding and improving the sensitivity of the assay. The use of perfusive matrix to perform a rapid flowthrough ELISA provides an improved method compared to the use of a well, because enzyme inhibitors which may be present in a sample are removed.

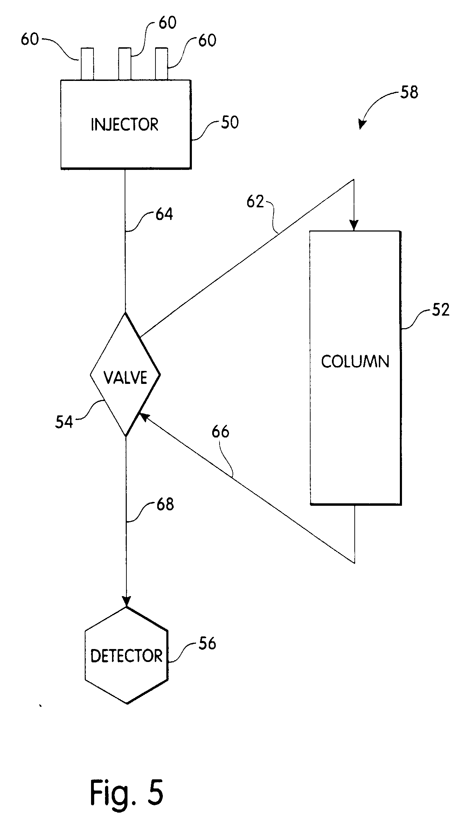

An apparatus 58 that may be utilized to perform an ELISA is shown in Figure 5. The apparatus 58 includes an injector 50, with entry ports 60 through which the analyte, tagging reagent or other samples are loaded. Sample liquids from injector 50 (e.g., a pump) are delivered through line 64 to a flow regulating valve 54. Valve 54 then directs flow through line 62 to column 52, containing close packed perfusive matrix particles comprising immobilized binding sites specific for the analyte. Flow then continues through line 66 back to valve 54, where the column eluent is diverted through line 68 to detector 56, e.g., a conventional spectrophotometer. The flow regulating valve 54 or the injector 50 may be used to provide a stop-flow mode on the column. In the stop-flow mode, flow from line 64 may be directed through valve 54 and then directly through line 68 to detector 56, or fluid flow may be interrupted by stopping injector 50. Assays may be readily performed using system 58. The stop-flow mode allows the flow to be stopped during the addition of any reagent, including the analyte or the tagging reagent, to enhance binding. The sensitivity of the assay can also be enhanced by mixing the analyte and the tagging reagent together in injector 50 before loading onto the column to further promote binding.

Perfusive matrix columns that may be utilized include Poros G/M (protein G affinity, low capacity 7 mg/ml-human IgG, 20 µm); Poros G/M (high capacity, 14 mg/ml human IgG, 20 µm); Poros R/M (reverse phase, 20 µm); and Poros OH/M (hydrophilic base material, 20µm), which are available from PerSeptive Biosystems, Inc. of Cambridge, Massachusetts, U.S.A.

### Example 1

An ELISA assay using IgG as the analyte was performed using a perfusive column on which IgG binding protein, protein G, was immobilized. A solution of a known concentration of IgG was perfused through the column, followed by a solution of the tagging reagent, an anti-IgG antibody linked to alkaline phosphatase. The IgG was assayed by perfusing the column with a solution of the enzyme substrate p-nitrophenyl phosphate (PNPP). The substrate solution was incubated by stopping the flow through the column for 30 seconds, and detecting the reaction by measuring the absorbance of column eluant at 450 nm.

The following reagents were used in the assay. The perfusive matrix column was Poros G/M (protein G affinity, low capacity, 7 mg/ml-human IgG, 20 µm), 0.1 x 2 cm, packed at 50 bar. The loading buffer was 1 M NH₄CH₃COOH, pH 7.1, containing 100 ug/ml bovine serum albumin (BSA) (Sigma, A-7906). The analyte, polyclonal goat-IgG (G-IgG) (Sigma, I-5256) was dissolved in loading buffer and syringe-filtered (Filtration Devices Inc., Acton, MA, 25 mm x 0.22 um), and loaded at 0.05 ml/min. Rabbit anti-goat F(ab)₂ tagged with alkaline phosphatase (RAG-AP) (Pierce, 31324) was diluted in loading buffer and syringe filtered and loaded at 0.01 ml/min. The enzyme substrate p-nitrophenyl phosphate (PNPP, Pierce, 34046) was dissolved in 10 mM diethanolamine, 0.5 mM MgCl₂, pH 9.5 at 0.1 or 1.0 mg/m. The absorbance was read at 405 nm after 30 seconds of stop flow. The solution was prepared fresh daily then filtered (Millipore, Type GV, 0.22 µm).

An HP1090 (Hewlett Packard) HPLC was utilized modified with a 10-port valve (Valco, Houston, TX). Samples were injected via an auto injector. An external valve switch diverted the flow over the column to the detector (Perkin-Elmer, LC-95). In the stop-flow mode, an external valve switch was activated which diverted the flow away from the column and to the detector. The stop-flow mode allowed substrate to be stopped on the column for a given length of time while, at the same time, taking a continuous background measurement of substrate alone. The column was stripped after several stop-flow time points were taken for each sample.

After each assay run, the protein G column was washed with loading buffer followed by a stripping solution (20 % acetic acid containing 300 mM MgCl₂) to remove all IgG, then reequilibrated with loading buffer. After this, PNPP was passed over the stripped column, before the next sample was added, and stop-flow measurements were taken at several time points to determine residual enzyme activity. If the background was too high the column was rewashed and the background was checked again.

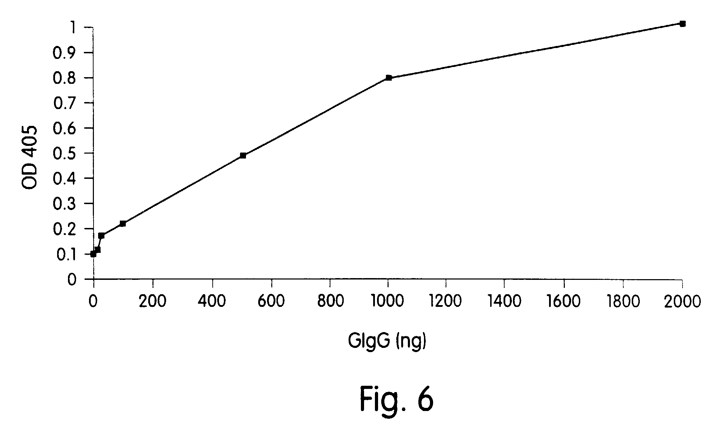

The results of the G-IgG assay are shown plotted in Fig. 6. As shown, optical density of the enzyme substrate solution is proportional to the number of nanograms of IgG present in the test solutions.

### Example 2

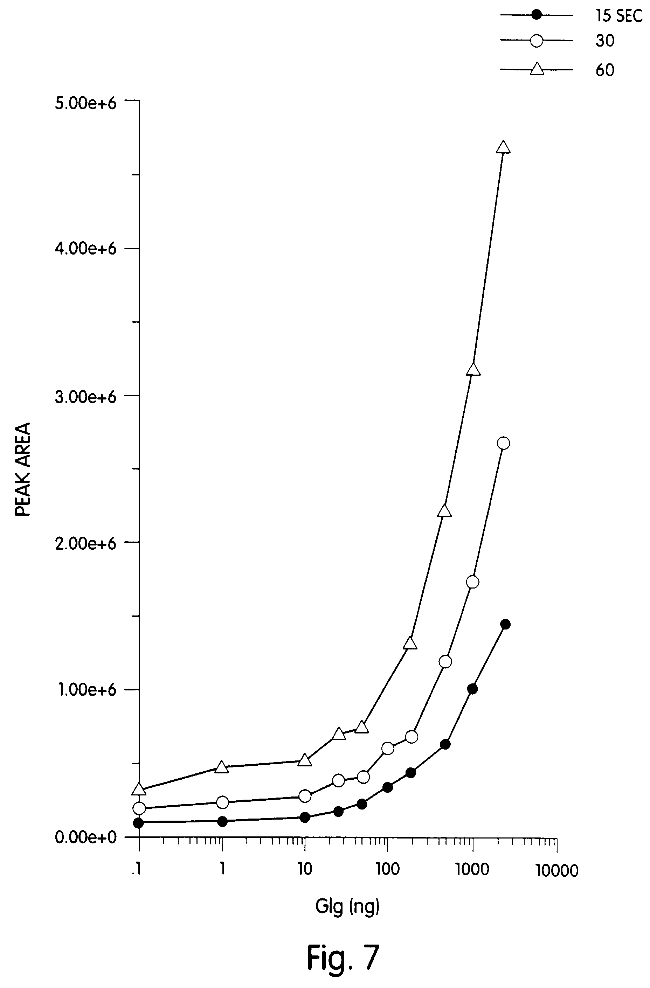

The procedure of Example 1 was followed, except that after loading the tagging reagent, rabbit anti-Goat F(ab)₂ alkaline phosphatase, the flow was stopped for 15, 30 and 60 seconds, respectively, to allow enhanced binding of the tagging reagent to the G-IgG on the column. PNPP was then loaded and the peak areas generated by enzyme product formation were analyzed using Maxima 820 software. As shown in Figure 7, the increase in residence time of the tagging reagent on the column can increase sensitivity of the assay, down to about 50 ng.

### Example 3

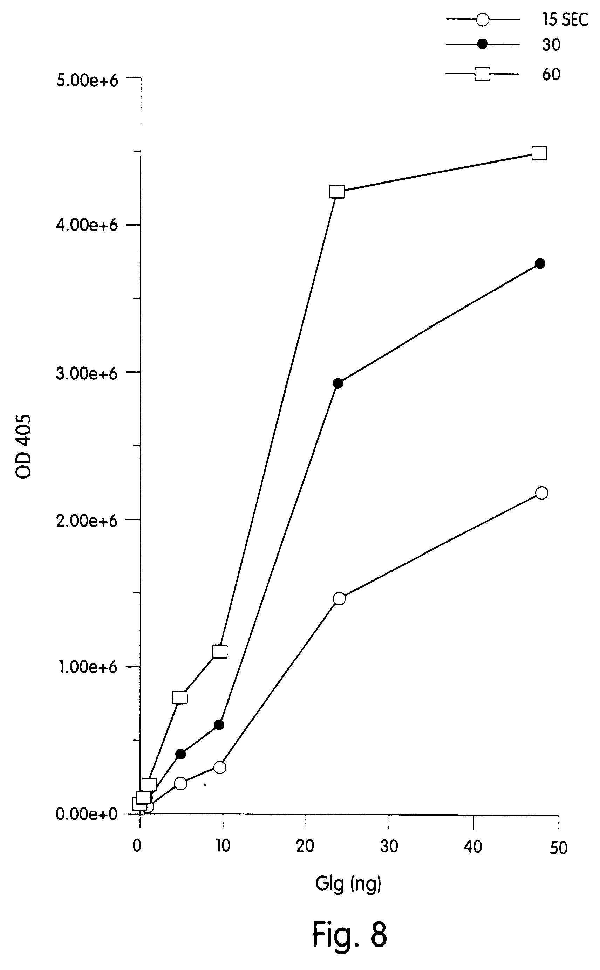

The assay procedure of Example 2 was followed, except that the IgG and the tagging reagent, rabbit anti-Goat F(ab)₂ alkaline phosphatase, were preincubated for 30 minutes at room temperature to facilitate binding, and then loaded onto the column. Enzyme substrate PNPP was then loaded onto the column with stop flow for 15, 30, or 60 seconds. The peak areas generated upon enzyme product formation were analyzed using Maxima 820 software. According to this method the sensitivity was further increased to 1 ng of analyte. The results are shown plotted in Figure 8.

## Claims

1. An apparatus for rapid flowthrough assay of an analyte, comprising:
a flowpath defined by a rigid matrix contained within a containing volume (52), the matrix having a surface with an area, the flowpath having a flowpath volume fillable with fluid having access to the surface,
first binding sites which are disposed on the surface and which bind selectively with an analyte to form a surface-immobilized binding site:analyte complex,
means for delivering convectively, to the flowpath volume, and substantially adjacent the surface, a liquid sample containing an analyte under conditions to permit formation of the binding site:analyte complex;
means for delivering convectively, to the flowpath volume, and substantially adjacent the surface, a detectable tagging reagent which binds with one of the binding site and the binding site:analyte complex, thereby to immobilize molecules of the tagging reagent on the surface, and
means (56) for detecting the quantity of the tagging reagent immobilized on the surface thereby to permit determination of the presence or concentration of analyte in the sample,
the ratio of the area of the surface to the flowpath volume being at least 10⁴ cm⁻¹.

2. The apparatus of claim 1 wherein said ratio is at least 10⁶ cm⁻¹.

3. The apparatus of claim 1, wherein the means for convective delivery of the analyte or the tagging reagent includes means for temporarily stopping flowthrough to enhance binding.

4. The apparatus of claim 1, wherein the tagging reagent binds with the analyte,
wherein the apparatus further comprises means for preincubating the analyte and the tagging reagent to allow binding of the analyte and the tagging reagent, and wherein the means for delivering the analyte and the means for delivering the tagging reagent are comprised of a single means for delivering convectively the bound analyte and tagging reagent together.

5. The apparatus of claim 1, wherein the means for detecting comprises a spectrophotometer, a spectrofluorimeter, or an illuminescent detector mounted for analysis of liquid exiting the matrix.

6. The apparatus of claim 1 wherein the tagging reagent comprises an enzyme, the apparatus further comprising means for flowing an enzyme substrate solution through the matrix, the means for detecting comprising means for detecting the product of the enzymatic reaction of the substrate.

7. The apparatus of claim 1 wherein the matrix comprises a microcapillary tube having an internal diameter less than about four micrometers.

8. The apparatus of claim 1 wherein the matrix comprises a plurality of intercontacted particles which define intraparticle throughpores and interparticle throughpores defining the flowpath through which the liquid sample or the tagging reagent is delivered.

9. The apparatus of claim 8 wherein the particles comprise interadhered clusters of smaller nonporous particles and the surface comprises the surface of the nonporous particles.

10. The apparatus of claim 1 wherein the surface comprises a hydrophobic polymer surface.

11. The apparatus of claim 10 wherein the binding protein is disposed on the surface by hydrophobic/hydrophobic interaction.

12. The apparatus of claim 8 wherein the surface comprises a cross-linked acrylic polymer surface.

13. The apparatus of claim 12 wherein the acrylic polymer comprises polystyrene.

14. The apparatus of claim 10 wherein said polymer comprises polystyrene divinyl benzene.

15. The apparatus of claim 1 wherein the matrix comprises a plurality of intercontacted nonporous particles which define interparticle throughpores having a mean diameter less than about four micrometers.

16. The apparatus of claim 15 wherein the particles comprise substantially spherical particles having an average diameter less than about 12 micrometers.

17. A rapid assay method comprising the steps of:
providing a flowpath defined by a rigid matrix contained within a containing volume (52), the matrix having a surface with an area, the flowpath having a flowpath volume fillable with fluid having access to the surface, the surface having a multiplicity of first binding sites which bind selectively with an analyte to form a surface-immobilized binding site:analyte complex, the ratio of the area to the flowpath volume being at least 10⁴ cm⁻¹,
delivering convectively, to the flowpath volume, and substantially adjacent the surface, a liquid sample containing an analyte to form a binding site:analyte complex,
delivering convectively, to the flowpath volume, and substantially adjacent the surface, a detectable tagging reagent which binds with one of the binding sites and the binding site:analyte complex, thereby to immobilize molecules of the tagging reagent on the surface, and
detecting the quantity of the tagging reagent immobilized on the surface, thereby to permit determination of the presence or the concentration of analyte in the sample.

18. The method of claim 17 wherein the ratio is at least 10⁶ cm⁻¹.

19. The method of claim 17 wherein the step of delivering convectively the analyte or the tagging reagent includes the step of temporarily stopping flowthrough to enhance binding.

20. The method of claim 17 wherein the tagging reagent binds with the analyte; wherein the method further comprises, prior to the step of delivering the analyte and the tagging reagent, preincubating the analyte and the tagging reagent together to enhance binding, and wherein the bound analyte and tagging reagent are delivered convectively together.

21. The method of claim 17 wherein the tagging reagent is detected by means of a spectrophotometer, a spectrofluorimeter, or an illuminescent detector mounted for analysis of liquid exiting the matrix.

22. The method of claim 17 wherein the tagging reagent comprises an enzyme, wherein the method further comprises providing a means for flowing an enzyme substrate solution through the matrix, and means for detecting the product of enzymatic reaction of the substrate.

23. The method of claim 17 wherein the matrix comprises a microcapillary tube having an internal diameter less than about four micrometers.

24. The method of claim 17 wherein the matrix comprises a plurality of intercontacted particles which define intraparticle throughpores and interparticle throughpores defining the flowpath through which the liquid sample or the tagging reagent is delivered.

25. The method of claim 24 wherein the particles comprise interadhered clusters of smaller nonporous particles and the surface comprises the surface of the nonporous particles.

## Patentansprüche

1. Vorrichtung zur schnellen Bestimmung einer zu analysierenden Substanz im Durchflußverfahren,
die aufweist:
- einen Strömungsweg, der durch eine nichtelastische Matrix vorgegeben ist, die in einem Behältervolumen (52) enthalten ist und eine Oberfläche mit einer Fläche aufweist, und der ein Strömungswegvolumen besitzt, das mit Flüssigkeit füllbar ist, die Zugang zur Oberfläche hat,
- erste Bindungsstellen, die sich auf der Oberfläche befinden und mit einer zu analysierenden Substanz eine selektive Bindung eingehen, wobei ein Komplex zwischen der an der Oberfläche immobilisierten Bindungsstelle und der zu analysierenden Substanz gebildet wird,
- eine Zufuhreinrichtung zur konvektiven Zufuhr einer flüssigen Probe, die eine zu analysierende Substanz enthält, zu dem Strömungswegvolumen und im wesentlichen in der Nachbarschaft der Oberfläche unter Bedingungen, unter denen sich der Komplex aus Bindungsstelle und zu analysierender Substanz bilden kann,
- eine Zufuhreinrichtung zur konvektiven Zufuhr eines erfaßbaren Markierungsreagens, das eine Bindung mit einer Bindungsstelle oder mit dem Komplex aus Bindungsstelle und zu analysierender Substanz eingeht, zu dem Strömungswegvolumen und im wesentlichen in der Nachbarschaft der Oberfläche unter Immobilisierung von Molekülen des Markierungsreagens an der Oberfläche
und
- eine Erfassungseinrichtung (56) zur Erfassung der Menge des an der Oberfläche immobilisierten Markierungsreagens, um so das Vorliegen oder die Konzentration der zu analysierenden Substanz in der Probe bestimmen zu können,
wobei das Verhältnis der Fläche der Oberfläche zum Strömungswegvolumen mindestens 10⁴ cm⁻¹ beträgt.

2. Vorrichtung nach Anspruch 1, wobei das Verhältnis der Fläche der Oberfläche zum Strömungswegvolumen mindestens 10⁶ cm⁻¹ beträgt.

3. Vorrichtung nach Anspruch 1, wobei die Zufuhreinrichtung zur konvektiven Zufuhr der zu analysierenden Substanz oder die Zufuhreinrichtung zur konvektiven Zufuhr des Markierungsreagens eine Einrichtung zur vorübergehenden Unterbrechung des Durchflusses zur Förderung der Bindung aufweist.

4. Vorrichtung nach Anspruch 1, wobei das Markierungsreagens eine Bindung mit der zu analysierenden Substanz eingeht,
die ferner eine Einrichtung zur Vorinkubation der zu analysierenden Substanz mit dem Markierungsreagens aufweist, um die Bindung der zu analysierenden Substanz an das Markierungsreagens zu ermöglichen,
und bei der die Zufuhreinrichtung zur Zufuhr der zu analysierenden Substanz und die Zufuhreinrichtung zur Zufuhr des Markierungsreagens aus einer einzigen Zufuhreinrichtung zur gemeinsamen konvektiven Zufuhr der gebundenen zu analysierenden Substanz und des Markierungsreagens bestehen.

5. Vorrichtung nach Anspruch 1, wobei die Erfassungseinrichtung ein Spektralphotometer, ein Fluoreszenzspektrometer oder ein Lumineszenzdetektor ist, die zur Analyse von aus der Matrix austretender Flüssigkeit angeordnet sind.

6. Vorrichtung nach Anspruch 1, wobei das Markierungsreagens ein Enzym ist, die ferner eine Einrichtung zum Durchleiten einer Enzymsubstratlösung durch die Matrix aufweist und bei der die Erfassungseinrichtung eine Einrichtung zur Erfassung des Produkts der enzymatischen Umsetzung des Substrats aufweist.

7. Vorrichtung nach Anspruch 1, wobei die Matrix ein Mikrokapillarröhrchen umfaßt, das einen Innendurchmesser von weniger als etwa 4 µm aufweist.

8. Vorrichtung nach Anspruch 1, wobei die Matrix aus einer Vielzahl miteinander in Kontakt stehender Partikel besteht, die innerhalb der Partikel vorliegende und zwischen den Partikeln vorliegende Durchgangsporen vorgeben, die ihrerseits den Strömungsweg vorgeben, durch den die flüssige Probe oder das Markierungsreagens zugeführt werden.

9. Vorrichtung nach Anspruch 8, wobei die Partikel Cluster aneinander haftender kleinerer nichtporöser Partikel sind und die Oberfläche die Oberfläche der nichtporösen Partikel ist.

10. Vorrichtung nach Anspruch 1, wobei die Oberfläche eine hydrophobe Polymeroberfläche ist.

11. Vorrichtung nach Anspruch 10, wobei ein zur Bindung befähigtes Protein durch hydrophobe Wechselwirkung an der Oberfläche angeordnet ist.

12. Vorrichtung nach Anspruch 8, wobei die Oberfläche eine vernetzte Acrylpolymer-Oberfläche umfaßt.

13. Vorrichtung nach Anspruch 12, wobei das Acrylpolymer Polystyrol enthält.

14. Vorrichtung nach Anspruch 10, wobei das Polymer ein Polystyrol-Divinylbenzol-Copolymer ist.

15. Vorrichtung nach Anspruch 1, wobei die Matrix aus einer Vielzahl miteinander in Kontakt stehender nichtporöser Partikel besteht, die zwischen den Partikeln liegende Durchgangsporen vorgeben, die eine mittlere Teilchengröße von weniger als etwa 4 µm aufweisen.

16. Vorrichtung nach Anspruch 15, wobei die Partikel im wesentlichen sphärische Partikel einer mittleren Teilchengröße von weniger als etwa 12 µm sind.

17. Schnelles Analysenverfahren, das folgende Schritte umfaßt:
- Vorsehen eines Strömungswegs, der durch eine nichtelastische Matrix vorgegeben ist, die in einem Behältervolumen (52) enthalten ist und eine Oberfläche mit einer Fläche aufweist, und der ein Strömungswegvolumen besitzt, das mit Flüssigkeit füllbar ist, die Zugang zur Oberfläche hat,
wobei die Oberfläche eine Vielzahl erster Bindungsstellen aufweist, die mit einer zu analysierenden Substanz eine selektive Bindung eingehen, wobei ein Komplex zwischen der an der Oberfläche immobilisierten Bindungsstelle und der zu analysierenden Substanz gebildet wird und das Verhältnis der Fläche der Oberfläche zum Strömungswegvolumen mindestens 10⁴ cm⁻¹ beträgt,
- konvektive Zufuhr einer flüssigen Probe, die eine zu analysierende Substanz enthält, zu dem Strömungswegvolumen und im wesentlichen in der Nachbarschaft der Oberfläche unter Bildung eines Komplexes aus Bindungsstelle und zu analysierender Substanz,
- konvektive Zufuhr eines erfaßbaren Markierungsreagens, das eine Bindung mit einer Bindungsstelle oder mit dem Komplex aus Bindungsstelle und zu analysierender Substanz eingeht, zu dem Strömungswegvolumen und im wesentlichen in der Nachbarschaft der Oberfläche unter Immobilisierung von Molekülen des Markierungsreagens an der Oberfläche
und
- Erfassung der Menge des an der Oberfläche immobilisierten Markierungsreagens, um so das Vorliegen oder die Konzentration der zu analysierenden Substanz in der Probe bestimmen zu können.

18. Verfahren nach Anspruch 17, wobei das Verhältnis der Fläche der Oberfläche zum Strömungswegvolumen mindestens 10⁶ cm⁻¹ beträgt.

19. Verfahren nach Anspruch 17, wobei der Schritt der konvektiven Zufuhr der zu analysierenden Substanz oder der Zufuhr des Markierungsreagens den Schritt der vorübergehenden Unterbrechung des Durchflusses zur Förderung der Bindung umfaßt.

20. Verfahren nach Anspruch 17, wobei das Markierungsreagens eine Bindung mit der zu analysierenden Substanz eingeht,
das ferner vor dem Schritt der Zufuhr der zu analysierenden Substanz und des Markierungsreagens eine Vorinkubation der zu analysierenden Substanz mit dem Markierungsreagens umfaßt, um deren Bindung zu fördern, und bei dem die gebundene zu analysierende Substanz und das Markierungsreagens gemeinsam konvektiv zugeführt werden.

21. Verfahren nach Anspruch 17, wobei das Markierungsreagens mit einem Spektralphotometer, einem Fluoreszenzspektrometer oder einem Lumineszenzdetektor bestimmt wird, die zur Analyse der aus der Matrix austretenden Flüssigkeit angeordnet sind.

22. Verfahren nach Anspruch 17, wobei das Markierungsreagens ein Enzym ist, bei dem ferner eine Einrichtung zum Durchleiten einer Enzymsubstratlösung durch die Matrix und eine Einrichtung zur Erfassung des Produkts der enzymatischen Umsetzung des Substrats eingesetzt werden.

23. Verfahren nach Anspruch 17, wobei die Matrix ein Mikrokapillarröhrchen umfaßt, das einen Innendurchmesser von weniger als etwa 4 µm aufweist.

24. Verfahren nach Anspruch 17, wobei die Matrix aus einer Vielzahl miteinander in Kontakt stehender Partikel besteht, die innerhalb der Partikel vorliegende und zwischen den Partikeln vorliegende Durchgangsporen vorgeben, die ihrerseits den Strömungsweg vorgeben, durch den die flüssige Probe oder das Markierungsreagens zugeführt werden.

25. Verfahren nach Anspruch 24, wobei die Partikel Cluster aneinander haftender kleinerer nichtporöser Partikel sind und die Oberfläche die Oberfläche der nichtporösen Partikel ist.

## Revendications

1. Appareil pour l'analyse rapide par écoulement d'une substance à analyser, comprenant:
une voie d'écoulement définie par une matrice rigide contenue dans un volume (52), la matrice ayant une superficie avec une surface, la voie d'écoulement ayant un volume de voie d'écoulement pouvant se remplir avec un fluide ayant accès à la superficie,
des premiers sites de liaison disposés sur la superficie qui se lient de façon sélective avec une substance à analyser en formant un complexe entre le site de liaison et la substance à analyser immobilisé à la superficie,
des moyens d'alimentation pour la délivrance convective d'un échantillon liquide contenant une substance à analyser au volume de voie d'écoulement et substantiellement adjacent à la superficie, sous des conditions permettant la formation du complexe site de liaison/substance à analyser,
des moyens d'alimentation pour la délivrance convective d'un réactif marqueur détectable qui se lie soit avec un site de liaison soit avec le complexe site de liaison/substance à analyser au volume de voie d'écoulement et substantiellement adjacent à la superficie de façon à immobiliser des molécules du réactif marqueur sur la superficie et
des moyens de détection (56) pour détecter la quantité du réactif marqueur immobilisé sur la superficie de façon à permettre la détection de la présence ou de la concentration en substance à analyser dans l'échantillon,
le rapport entre la surface de la superficie et le volume de la voie d'écoulement étant d'au moins 10⁴ cm⁻¹.

2. Appareil selon la revendication 1, dans lequel le rapport est d'au moins 10⁶ cm⁻¹.

3. Appareil selon la revendication 1, dans lequel les moyens d'alimentation pour la délivrance convective de la substance à analyser ou du réactif marqueur comprenant des moyens pour arrêter temporairement l'écoulement dans le but de favoriser la liaison.

4. Appareil selon la revendication 1, dans lequel le réactif marqueur se lie avec la substance à analyser, l'appareil comprend de plus des moyens pour la préincubation de la substance à analyser avec le réactif marqueur pour permettre la liaison entre la substance à analyser et le réactif marqueur, et les moyens d'alimentation pour la délivrance de la substance à analyser et les moyens d'alimentation pour la délivrance du réactif marqueur consistent en un seul moyen d'alimentation pour la délivrance convective de la substance à analyser et du réactif marqueur ensemble.

5. Appareil selon la revendication 1, dans lequel les moyens de détection comprennent un spectrophotomètre, un spectrofluoromètre ou un détecteur de luminescence prévus pour l'analyse d'un liquide sortant de la matrice.

6. Appareil selon la revendication 1, dans lequel le réactif marqueur est une enzyme, l'appareil comprend de plus des moyens pour l'écoulement d'une solution de substrat de l'enzyme au travers de la matrice, les moyens de détection comprenant des moyens de détection du produit de la réaction enzymatique du substrat.

7. Appareil selon la revendication 1, dans lequel la matrice comprend un tube microcapillaire ayant un diamètre interne inférieur à environ 4 µm.

8. Appareil selon la revendication 1, dans lequel la matrice consiste en une multitude de particules inter-connectées qui définissent des pores continus intra-particules et des pores continus interparticules définissant la voie d'écoulement au travers desquels l'échantillon liquide ou le réactif marqueur sont délivrés.

9. Appareil selon la revendication 8, dans lequel les particules sont des pelotons interadhérés de particules non-poreuses plus petites, et la superficie est la superficie des particules non-poreuses.

10. Appareil selon la revendication 1, dans lequel la superficie est une superficie de polymère hydrophobe.

11. Appareil selon la revendication 10, dans lequel une protéine capable de se lier est prévue sur la superficie par interaction hydrophobe.

12. Appareil selon la revendication 8, dans lequel la superficie comprend une superficie de polymère acrylique réticulé.

13. Appareil selon la revendication 12, dans lequel le polymère acrylique comprend du polystyrène.

14. Appareil selon la revendication 10, dans lequel le polymère est un copolymère polystyrène/divinylbenzène.

15. Appareil selon la revendication 1, dans lequel la matrice consiste en une multitude de particules non-poreuses étant en contact entre elles et définissant des pores continus interparticules ayant un diamètre moyen inférieur à environ 4 µm.

16. Appareil selon la revendication 15, dans lequel les particules sont des particules substantiellement spériques ayant un diamètre moyen inférieur à environ 12 µm.

17. Procédé d'analyse rapide comprenant les étapes suivantes:
- prévoir une voie d'écoulement définie par une matrice rigide contenue dans un volume (52), la matrice ayant une superficie avec une surface, la voie d'écoulement ayant un volume de voie d'écoulement pouvant se remplir avec un fluide ayant accès à la superficie, la superficie ayant une multitude de premiers sites de liaison qui se lient de façon sélective avec une substance à analyser en formant un complexe site de liaison/substance à analyser immobilisée à la superficie, le rapport entre la surface et le volume d'écoulement étant au moins de 10⁴ cm⁻¹,
- à délivrer convectivement un échantillon liquide contenant une substance à analyser au volume de voie d'écoulement et adjacent à la superficie en formant un complexe site de liaison/substance à analyser,
- à délivrer convectivement un réactif marqueur détectable qui se lie soit avec un site de liaison soit avec le complexe site de liaison/substance à analyser au volume de voie d'écoulement et adjacent à la superficie de façon à immobiliser des molé cules du réactif marqueur sur la superficie, et
- à détecter la quantité de réactif marqueur immobilisé sur la superficie, de façon à permettre la détection de la présence ou de la concentration en substance à analyser dans l'échantillon.

18. Procédé selon la revendication 17, dans lequel le rapport est d'au moins 10⁶ cm⁻¹.

19. Procédé selon la revendication 17, dans lequel l'étape de la délivrance convective de la substance à analyser ou du réactif marqueur comprend l'étape d'arrêt de façon temporaire de la voie d'écoulement pour favoriser la liaison.

20. Procédé selon la revendication 17, dans lequel le réactif marqueur se lie avec la substance à analyser, et le procédé comprend de plus, avant l'étape de la délivrance de la substance à analyser et du réactif marqueur, la préincubation de la substance à analyser avec le réactif marqueur pour favoriser la liaison, et la substance à analyser et le réactif marqueur liés sont délivrés ensemble convectivement.

21. Procédé selon la revendication 17, dans lequel le réactif marqueur est détecté au moyen d'un spectrophotomètre, d'un spectrofluoromètre ou d'un détecteur de luminescence prévus pour l'analyse du liquide sortant de la matrice.

22. Procédé selon la revendication 17, dans lequel le réactif marqueur est une enzyme, et le procédé comprend de plus des moyens pour l'écoulement d'une solution de substrat de l'enzyme au travers de la matrice et des moyens pour détecter le produit de la réaction enzymatique du substrat.

23. Procédé selon la revendication 17, dans lequel la matrice comprend un tube microcapillaire ayant un diamètre interne inférieur à environ 4 µm.

24. Procédé selon la revendication 17, dans lequel la matrice consiste en une multitude de particules inter-connectées qui définissent des pores continus intra-particules et des pores continus interparticules définissant la voie d'écoulement au travers desquels l'échantillon liquide ou le réactif marqueur sont délivrés.

25. Procédé selon la revendication 24, dans lequel les particules sont des pelotons interadhérés de particules non-poreuses plus petites, et la superficie est la superficie des particules non-poreuses.
